# EUROPEAN PATENT APPLICATION

(11) **EP 1 421 960 A1**
(43) Date of publication of application: **26.05.2004**
(21) Application number: 03025258.9
(22) Date of filing: 06.11.2003
(51) Int. Cl.: A61M 5/165

(54) **Infusion filter operating in various tridimensional positions**

(30) Priority: 21.11.2002 IT MI20022473
(71) Applicant: GVS S.p.A., 40069 Zola Predosa (Bologna) (IT)
(72) Inventor: Scagliarini, Massimo, 40136 Bologna (IT); Bettini, Emanuele, 40050 Monte San Pietro (Bologna) (IT)
(74) Representative: Ripamonti, Enrico, Dr. Ing.

(57) **Abstract**

A filter (1) comprises a box casing (2) in which at least one cavity (37) is present between an outer element (3, 4) of said casing (2) and an inner surface (5A, 5B) presenting a plurality of channels (21) on which a corresponding hydrophilic filtering membrane (30) lies, said cavity (37) communicating with a conduit (27) for entry of the fluid into the filter (1) and said channels (21) being connected to a conduit (23) for exit of said fluid, in said outer element (3, 4) there being provided through apertures (40, 41) with which hydrophobic membranes (44) are associated. A surface (S1) bounded by the shortest possible ideal closed line (70), which totally comprises all the hydrophobic membranes (44), contains substantially within its interior the projection thereon of the useful hydrophilic surface (S2) of the hydrophilic filtering membrane (30), this enabling the filter (1) to be employed in a plurality of spatial positions during its use.

## Description

The present invention relaters to an infusion filter in accordance with the introduction to the main claim.

Filters of the same type as the present invention have been known for some time. They present small dimensions, but must be used in well defined spatial orientations to prevent the formation of air bubbles within the filter which, if they should reach the patient by being conveyed by the fluid, would result in serious well-known problems.

An object of the present invention is to provide an infusion filter which is improved with respect to similar known filters.

A particular object of the invention is to provide a filter of the stated type which during use can be disposed in a multiplicity of spatial positions without this involving risks to the correct fluid flow to a patient.

Another object is to provide a filter of the stated type which can be used reliably and safely.

These and other objects which will be apparent to the expert of the art are attained by a filter in accordance with the accompanying claims.

The present invention will be more apparent from the accompanying drawing, which is provided by way of non-limiting example and in which:
Figure 1 is a front view of a filter according to the invention;
Figure 2 is a side view of the filter of Figure 1;
Figure 3 is a section on the line 3-3 of Figure 1;
Figure 4 is an exploded view of the filter of Figure 1;
Figure 5 is a schematic view of a characteristic of Figure 1;
Figure 6 is a perspective view of a variant of the filter of Figure 1;
Figure 7 is a partial perspective view of another variant of the filter of Figure 1;
Figure 8 is a section on the line 8-8 of Figure 7;
Figure 9 is a partial perspective view of a further variant of the filter of Figure 1;
Figure 10 is a section on the line 10-10 of Figure 9;
Figure 11 is a partial perspective view of another variant of the filter of Figure 1;
Figure 12 is a section on the line 12-12 of Figure 11;
Figure 13 is a partial perspective view of another variant of the filter of Figure 1;
Figure 14 is a section on the line 14-14 of Figure 13;
Figure 15 is a perspective view from above of a further variant of the filter of Figure 1; and
Figure 16 is a section on the line 16-16 of Figure 15.

With reference to said Figures from 1 to 14, a filter according to the invention is indicated overall by 1 and comprises a box casing 2 defined, in the example under examination, by a first and a second outer element 3, 4 closing an intermediate element 5. These box casing elements 3, 4 and 5 are constructed preferably of plastic material in any known manner.

The outer elements 3 and 4 comprise a flat portion 6 and 7 having opposing faces 6A, 6B and 7A, 7B respectively. In proximity to the edge 9 of said portions 6, 7, there projects from their face 6B, 7B, which is internal with respect to the casing 2 (the face 6A and 7A being an external face of this latter), a shoulder 10 arranged to cooperate with a recess 11 provided in the facing surface of the element 5, in order to secure the elements 3 and 4 to the intermediate element 5. This fixing is obtained in any known manner, for example by ultrasonic bonding, gluing or other means.

The surface facing the face 6B is indicated in the figures by 5A, while the surface facing the face 7B is indicated by 5B.

Only one of the surfaces 5A and 5B is described hereinafter as these are identical. Likewise only one of the elements 3 and 4 is described hereinafter, it being understood that everything stated for the surface 5A and for the element 3 is also valid for the surface 5B and for the element 4.

The intermediate element 5 presents a rounded edge 16 and comprises on the face 5A, starting from its periphery and progressing towards its interior, a pair of spaced-apart parallel annular shoulders 17 and 18 defining the aforesaid recess 11, an annular step 19 and a plurality of parallel ribs 20, circumscribed by the step 19 and defining channels 21 closed at one end 21A by the step 19 and open at their other end 21B where they communicate with a conduit 23 leaving the element 5 via a stem 24 projecting from the edge 16 of said element.

The step 19 and the parallel ribs 20 have a height less than the shoulders 17 and 18. Between the step 19 and the shoulder 18 a cavity 26 is present communicating with an entry conduit 27 which penetrates into the element 5 (via the shoulders 17 and 18) by passing through a stem 29 projecting from the edge 16. Preferably the stem 29 is coaxial with the stem 24, they both lying along a central axis A of the element 5.

The step 19 and the ribs 20 can be formed directly in one piece with the element 5 or can be formed on a separate piece inserted within the shoulders 17 and 18 of the element 5 in such a manner as to rest along the shoulder 18 in correspondence with two of its side portions, but spaced from said shoulder 18 so as to define the cavity 26.

As stated, the free ends of the step 19 and of the ribs 20 lie at least in a plane distant from that in which the ends of the shoulders 17 and 18 lie. Within this space a hydrophilic filter membrane 30 is positioned to rest against the shoulder 18 but not to cover the cavity 26. In this respect, in correspondence with this latter, during filter assembly the hydrophilic membrane 30 is maintained distant from the shoulder 18 by cusp-shaped projections 33 jutting from this shoulder in correspondence with a transverse part 19A of the step 19 perpendicular to said axis A. The membrane is finally rested on the ends of the ribs 20 and is finally fixed to the transverse part 19A and to the step 10 in known manner, for example by hot bonding.

When the intermediate element 5 is completed (i.e. also provided with the membranes 30), cavities 37 communicating with the aforesaid cavities 26 are present between its faces 5A and 5B and the adjacent faces 6B and 7B of the outer elements 3 and 4.

Each outer element 3, 4 (also having a rounded edge 9 such as that of the element 5) comprises at least two through apertures 40 and 41 each provided in proximity to sides 42 and 43 of said element which are perpendicular to the axis A. A hydrophobic membrane 44 of known type is positioned in correspondence with each of these apertures.

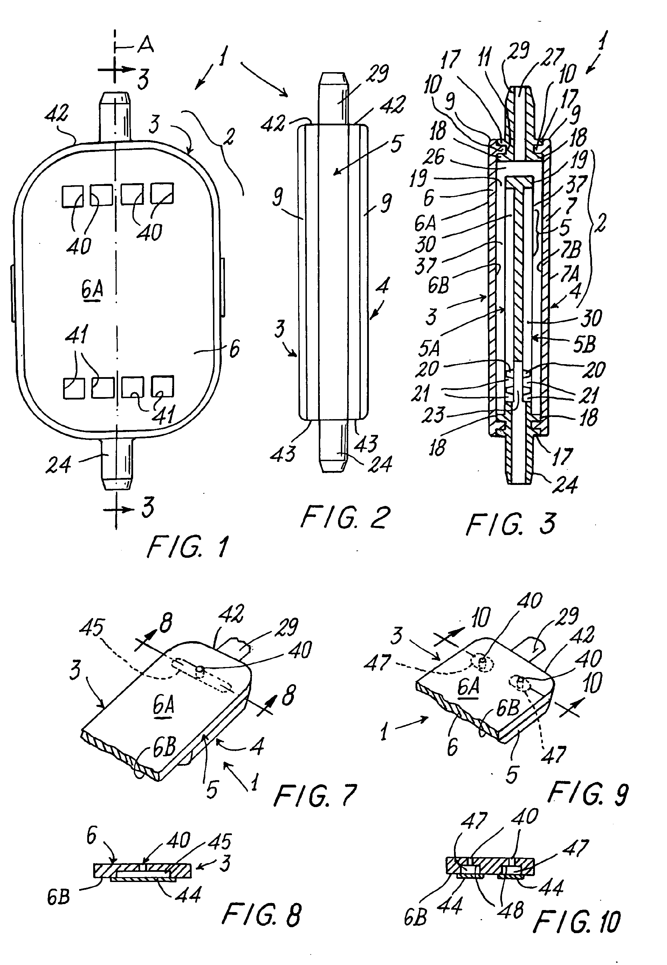

In particular, in Figures 1-4 each outer element 3, 4 comprises four apertures 40 and four adjacent apertures 41. However the number of these apertures can also be different: for example. in Figure 6, in correspondence with the sides 42 and 43 of the elements 3 and 4 a single aperture 40 and 41 is present having an evidently large transverse length. A single aperture 40 and 41 is also present in Figure 7, in proximity to said sides; however each of these apertures is connected to a large underlying recess 45 (rectangular in this example) provided within the interior face 6B of the portion 6 of the element 3 in correspondence with which a hydrophobic membrane 44 is present. In contrast, in Figure 9 in correspondence with each side 42 and 43 (only the side 42 is shown) a pair of apertures 40 and 41 are present, connected to an underlying recess 47 of larger dimensions provided within the face 6B of the portion 6; a step 48 is present between the aperture 40 (or 41) and the underlying recess 47, the hydrophobic membrane 44 being positioned in correspondence with this recess. In Figure 11, within the face 6B of the portion 6 of the element 3, in correspondence with each side 42 and 43, a substantially rectangular recess 50 is present, connected to two conduits 51 opening into the face 6A via corresponding apertures 40 (and 41). Finally in Figure 13, in the face 6B of the portion 6 (in proximity to the sides 42 and 43) a circular recess 53 is provided connected to the apertures 40 (or 41) via channels 54 with their axis inclined to the plane of the face 6A in which the apertures 40 (or 41) are located.

It should be noted that each membrane is preferably and advantageously associated with the relative aperture 40 or 41 or with the recess 45, 47, 50, 53 by being fixed to the face 6B of the part 3, rather than by being inserted into the respective hole or recess. This enables a filtering surface to be obtained which is larger than that obtained if the membrane were inserted into the corresponding hole or recess in that, in this latter case, a part of the useful volume of the membrane would be occupied by the bond between the membrane and the wall of the corresponding hole or recess.

The hydrophobic surfaces defined by the membrane 44 can be all connected together by a closed line 70, shown dashed in Figure 4 and full in Figure 5. Preferably, this line is the shortest which ideally connects together all the said surfaces of the membranes 44, i.e. it is defined by rectilinear portions in the example of the figures. According to the invention, this closed line defines a surface S1 within which the projection of the useful hydrophilic surface S2 of the underlying membrane 30 substantially falls, the "useful" surface meaning the effectively filtering surface of the membrane 30. The surface S1 is that enclosed by the line 70, the surface S2 being the hatched surface in Figures 4 and 5.

By virtue of this characteristic, after a usual line priming phase, proper filter effectiveness is achieved whatever its position in space during its use (vertical to, inclined to or parallel to an underlying plane). This is because the fluid entering the filter 1 is able to completely occupy the cavity 37 by expelling the air present therein and filtering through substantially the entire useful surface of the membrane (in the aforestated sense). In this manner, the filter is completely operative, and effective in filtering the entering fluid, in that substantially the entire useful surface of the hydrophilic membrane 30 (at most except for a peripheral portion) participates in the filtering. In addition, the channels 21 are completely filled by the fluid which filters through the membrane 30 such that from one end 21A (that facing the entry conduit 27) to their other end 21 B (that communicating with the exit conduit 23) they contain no residual air bubbles, with obvious positive implications for the fluid feed to the user.

It should be noted that at most, under utilization conditions, a possible minimum part of the useful surface S2 of the projection of the hydrophilic membrane 30 can lie outside the surface S1, provided that the geometry of the seat in which the membrane 30 is positioned enables the surface tension effect of the filtered fluid to be utilized, this effect occurring if the distance between the membrane 30 and the face 6B of the portion 6 (i.e. the depth of the cavity 37 measured perpendicular to the axis A) lies between 0.1 mm and 3 mm, preferably between 0.5 and 2 mm and advantageously between 0.5 mm and 1.5 mm. Under these conditions, the possible minimum (peripheral) surface part of the membrane 30, the projection of which does not fall within the surface S1, becomes in any event a fluid passage by capillary effect, with consequent complete use of the capacity of said membrane (i.e. the hydrophilic filtering surface of the membrane 30 is always 100% of its area).

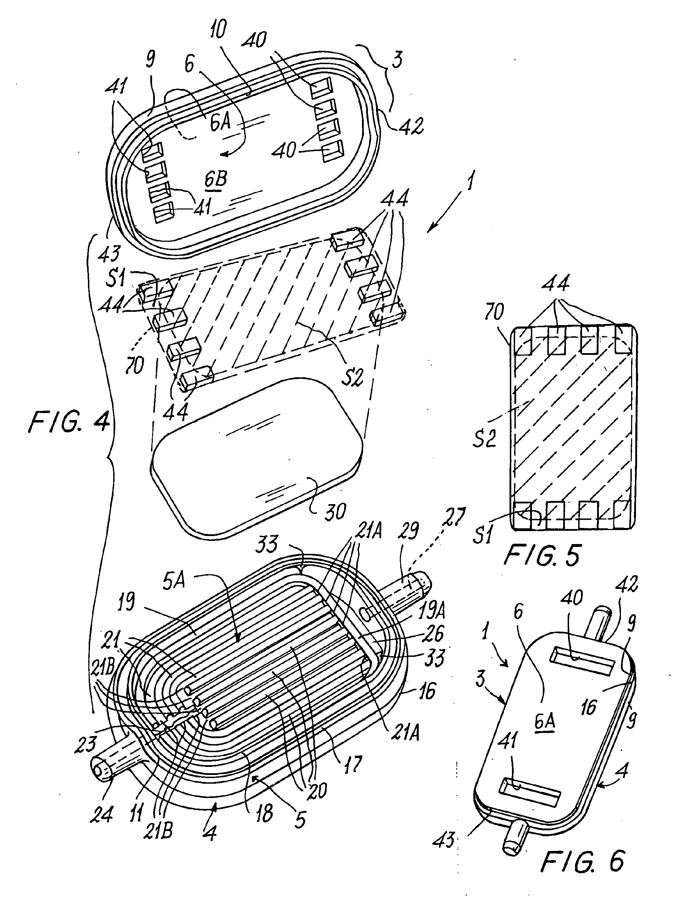

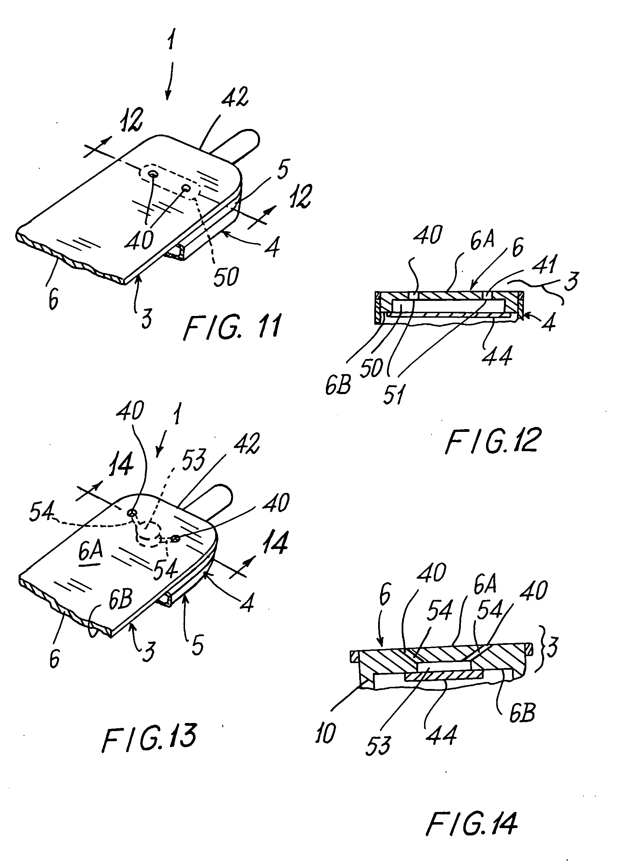

By virtue of the invention, the described and claimed filter presents high functional capacity, exceeding that of known filters. This is because of the arrangement of the apertures 40 and 41 provided with the hydrophobic membranes 44, by virtue of which a high functional capacity of the filtering surface is obtained; this is also due to the fact that these apertures cooperate directly (as in Figures 1-6) or indirectly (as in Figures 7-14) with membranes 44 of considerable area (even greater than that of said apertures, as in Figures 7-14), which ensure a high air flow from the casing 2 of the filter 1.

Other embodiments are evidently possible within the light of the present description, provided they remain within the scope of the accompanying claims. For example, each membrane 30 can be of any form, including complex (as can the arrangement of the underlying channels 21), the filter operating effectively provided the apertures present within the outer element 3 or 4 which face said membrane are such as to define, by means of the closed line which joins them together, the surface S1 with the aforedescribed characteristics. In the limit, a single aperture of large dimensions can be present in said element.

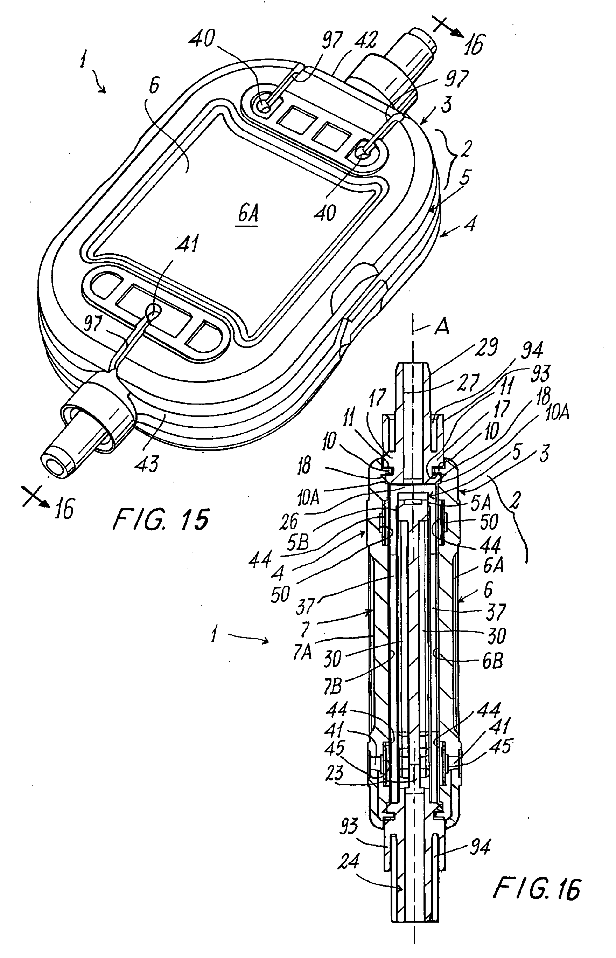

Another embodiment of the invention is shown in Figures 15 and 16 in which parts corresponding to those of the already described figures are indicated by the same reference numerals. In the figures under examination, the filter presents the apertures 40 and 41 connected to differently shaped recesses: the apertures 40 are associated with a recess 50 in accordance with the embodiment of Figure 11, while the aperture 41 is associated with a recess 45 in accordance with the embodiment of Figure 7.

The embodiment under examination also presents other differences with those already described; for example, in correspondence with the shoulder 17 and around the stems 24 and 29, the element 5 presents circular rims 93 spaced from the corresponding stems and defining therewith recesses 94 for accepting the end of a corresponding contact or tube connected to a vessel of liquid (for example physiological liquid), in the case of the stem 29, or connected to the patient in the case of the stem 24. The rim 93 is essentially a prolongation of the shoulder 17.

The shoulder 18 is originally formed of tapered shape (triangular in cross-section) such that when inserted into a recess 10A adjacent to the shoulder 10 and provided in each face 6B, 7B of the elements 3 and 4 towards the interior of the filter, it can be fused into this recess during for example the hot bonding, so securely joining the element 5 to the adjacent elements 3 and 4.

Finally, the apertures 40 and 41 are connected to recesses 97 formed in the external face 6A, 7A of the flat portions 6 and 7 to facilitate the escape of air from these apertures. These recesses lie parallel to the (longitudinal) axis A of the filter.

Said apertures, and those of the filter represented in the previously described figures, can be closed by suitable plugs (not shown) which can be maintained connected to the filter casing 2 (for example by a filiform connection element, for example of plastic material) or can be of the type completely separable from the filter. The purpose of these plugs is to prevent air being drawn from the outside into the filter interior when one of the tubes connected to the filter (in particular, that connected to a vessel of liquid) is subjected to vacuum caused for example by a syringe.

Embodiments in which the membrane element 5 presents two opposing surfaces 5A and 5B provided with channels 21 have been described and shown in the figures. However the scope of the present invention also comprises a filter in which this element 5 presents a single face (for example, 5A) provided with channels, whereas the other (the face 5B) is completely flat. In this case, the outer element 4 is not present and the face 5B of the element 5 closes the filter on the side opposite that on which the element 3 is present.

## Claims

1. A filter (1) for filtering a fluid directed towards a patient, comprising a box casing (2) in which at least one cavity (37) is present defined by an outer element (3, 4) of said casing (2) and an inner surface (5A, 5B) presenting a plurality of channels (21) on which a corresponding hydrophilic filtering membrane (30) lies, said cavity (37) communicating with a conduit (27) for entry of the fluid into the filter (1) and said channels (21) being connected to a conduit (23) for exit of said fluid from the filter (1), in said element (3, 4) of the box casing (2) there being provided spaced-apart through apertures (40, 41) close to its opposing ends (42, 43) and with which hydrophobic membranes (44) are associated, **characterised in that** a surface (S1) bounded by an ideal closed line (70), which totally comprises all the hydrophobic membranes (44), contains substantially within its interior the projection thereon of the useful hydrophilic surface (S2) of the hydrophilic filtering membrane (30), this enabling the filter (1) to be employed in a plurality of spatial positions during its use.

2. A filter as claimed in claim 1, **characterised in that** the closed line bounding the surface (S1) comprising the hydrophobic membranes (44) is the shortest line which joins these latter together.

3. A filter as claimed in claim 1, **characterised in that** the distance between said element (3, 4) of the box casing (2) and the hydrophilic filtering membrane (30) lies between 0.1 mm and 3 mm, preferably between 0.5 mm and 2 mm.

4. A filter as claimed in claim 3, **characterised in that** the distance between said element (3, 4) of the box casing (2) and the hydrophilic filtering membrane (30) lies between 0.5 mm and 1.5 mm.

5. A filter as claimed in claim 1, **characterised in that** the through apertures (40, 41) have a size identical to that of the membranes (44) associated with them.

6. A filter as claimed in claim 1, **characterised in that** the through apertures (40, 41) have a size less than that of the membranes (44) associated with them.

7. A filter as claimed in claim 6, **characterised in that** each membrane (44) is associated with a recess (45, 47, 50, 53) provided within a face (6B, 7B) of the element (3, 4) of the box casing (2) facing the hydrophilic membrane (30), with said recess (45, 47, 50, 53) there being associated at least one aperture (40, 41) opening into the opposing face (6A, 7A) of said element (3, 4), between said aperture and said recess there being present at least one step (48) so that the aperture has a size less than that of the recess.

8. A filter as claimed in claim 7, **characterised in that** the recess is of polygonal shape.

9. A filter as claimed in claim 7, **characterised in that** the recess is of circular shape.

10. A filter as claimed in claim 1, **characterised in that** each hydrophobic membrane (44) has a surface greater than that of the aperture (40, 41) with which it is associated.

11. A filter as claimed in claim 10, **characterised in that** the hydrophobic membrane is fixed to that face (6B, 7B) of the element (3, 4) of the box casing (2) facing the hydrophilic membrane (30), in correspondence with the relative aperture (40, 41).

12. A filter as claimed in claim 1, **characterised in that** the channels (21) of the inner surface (5A, 5B) present a closed end (21A) facing and close to the entry conduit (27), and the other end (21B) connected to the exit conduit (23).

13. A filter as claimed in claim 12, **characterised in that** the closed end (21 A) of said channels (21) is closed by an annular element (19) which surrounds said channels (21).

14. A filter as claimed in claim 1, **characterised in that** the entry conduit (27) and exit conduit (23) are provided within an element (5) of the box casing (2) presenting the surface (5A, 5B) with the channels (21) and connected to the outer element (3, 4) of said casing (2).

15. A filter as claimed in claim 14, **characterised in that** the entry conduit (27) and exit conduit (23) are provided within stems (29, 24) projecting from the box casing element (5) provided with channels (21).

16. A filter as claimed in claim 15, **characterised in that** around each stem (24, 29) an annular rim (93) is present defining with the corresponding stem (24, 29) a recess (94) for receiving the end of a corresponding conduit connected to the filter.

17. A filter as claimed in claim 14, **characterised in that** the element (5) with the surface (5A) provided with channels (21) presents a second surface (5B), opposing the surface (5A) with channels, but not provided with these latter.

18. A filter as claimed in claim 14, **characterised in that** the element (5) with the surface (5A) provided with channels (21) presents a second surface (5B), opposing the surface (5A) with channels (21) and shaped as this latter, to the front of said second surface (5B), also provided with channels (21) on which a hydrophilic membrane (30) is superposed, there being positioned a second outer element (4) of the box casing (2) provided with apertures (40, 41) with which hydrophobic membranes (44) are associated, between said second outer element (4) and the element (5) with the surfaces (5A., 5B) provided with channels (21) there being present a cavity (37) connected to the entry conduit (27), said element (5) with the surfaces (5A, 5B) provided with channels (21) being intermediate between the outer elements (3, 4) of the box casing (2).

19. A filter as claimed in claim 1, **characterised in that** the apertures (40, 41) are connected to recesses (97) provided in a free face (6A, 6B) of the corresponding outer element (3, 4).

20. A filter as claimed in claim 19, **characterised in that** the recesses (97) lie parallel to the longitudinal axis (A) of the filter.

21. A filter as claimed in claim 1, **characterised in that** the apertures (40, 41) cooperate with removable shut-off members.

22. A filter as claimed in claim 21, **characterised in that** the shut-off members are connected to the filter casing (2).

23. A filter as claimed in claim 22, **characterised in that** the shut-off members are completely separable from the filter casing (2).
